# EUROPEAN PATENT APPLICATION

(11) **EP 2 532 374 A2**
(43) Date of publication of application: **12.12.2012**
(21) Application number: 12275080.5
(22) Date of filing: 31.05.2012
(51) Int. Cl.: A61L 31/10, A61L 31/16

(54) **Method of manufacturing coating agent for drug releasing stent and coating agent for drug releasing stent manufactured thereby**

(30) Priority: 07.06.2011 KR 20110054540
(71) Applicant: Taewoong Medical Co., Ltd., Kimpo-si Kyonggi do 415-873 (KR); Shin, Kyong-Min, Seoul (KR)
(72) Inventor: Shin, Kyong-Min, Seoul (KR); Na, Kun, Kyongki-do (KR)
(74) Representative: McCartney, Jonathan William

(57) **Abstract**

This invention relates to a method of manufacturing a coating agent for a drug releasing stent and to a coating agent for a drug releasing stent manufactured thereby. This method includes (1) dissolving polyurethane in tetrahydrofuran, (2) dissolving pluronic F-127 in tetrahydrofuran, (3) dissolving a gemcitabine compound in ethanol, (4) mixing these three solutions obtained in (1) to (3) thus preparing a solution mixture, (5) applying the solution mixture obtained in (4) on a stent coated with a Teflon film, (6) drying the stent of (5) for a predetermined period of time and then immersing the stent in a polyurethane solution in tetrahydrofuran, and (7) removing the stent immersed in (6) from the polyurethane solution and then drying the stent, so that the rate of release of an anti-cancer drug applied on the stent can be continuously and maximally improved thereby maximizing anti-cancer efficacy.

## Description

### BACKGROUND OF THE INVENTION

### 1. Field of the Invention

The present invention relates to a method of manufacturing a coating agent for a drug releasing stent and a coating agent for a drug releasing stent manufactured thereby, and more particularly to a method of manufacturing a coating agent for a drug releasing stent, which is capable of maximizing the rate of release of a drug which is applied on a stent so as to exhibit anti-cancer efficacy, and to a coating agent for a drug releasing stent manufactured thereby.

### 2. Description of the Related Art

Typically, in surgical treatment or invasive drug treatment associated with such surgical treatment, stents (artificial wire meshes) are widely used to supply a support or prosthesis within blood vessels or lumina in order to prevent the recurrence of stenosis. Furthermore, to promote treatment or recovery, stents may be inserted into or may expand the esophagus, respiratory tract, blood vessels, urinary organs, or other lumina which are difficult to access, which is regarded as a general therapy.

Stents, which were developed in the late 1990s, may be applied instead of making an artificial anus when operating on patients suffering from colorectal cancer, but cases may exist where such stents cannot be used depending on the circumstances of the patients.

Stent implantation is not used to treat cancer but is used to temporarily solve the problem of a portion of the intestines being narrowed due to cancer.

A stent which is provided in the form of a thin metal tube is inserted into a portion in which the large intestine is narrowed due to cancer and expands it so as to keep the blocked portion open. Although many kinds of stents suitable for use in, for example, colorectal cancer, have been proposed, an alloy of nickel and titanium (which is referred to as "Nitinol") is particularly useful.

Recently, in order to improve therapeutic effects using stents, many attempts have been made to develop graft-stents for delivering drugs such as thrombolytic or antiproliferative agents. For example, US Patent No. 5,092,877 discloses a polymeric stent that may be used along with a coating material in relation to drug release, and International Patent Publication No. WO96/032907 discloses a drug release coated stent.

With the goal of coating the stent with a drug so as to transfer such a bioactive therapeutic material over a long period of time, methods have been devised, which include adding the bioactive therapeutic material to a polymer solution thus obtaining a mixture, which is then applied on a stent, followed by removing the solvent, so that a polymer layer containing the bioactive therapeutic material is formed on the stent.

The case where a specific bioactive therapeutic material, especially dexamethasone, is used has to take into consideration the miscibility with the solvent or polymers used or solubility therein, and the release rate should become appropriate.

Korean Patent No. 10-439156 discloses techniques for mixing a bioactive material selected from the group consisting of dexamethasone, paclitaxel, mitomycin and ibuprofen with a polymer material comprising 0.01 ~ 30 wt% of a water-soluble polymer coprecipitate and 70 ~ 99.99 wt% of a crosslinked polymer solution, and for coating a stent with this mixture.

Korean Patent No. 10-511618 discloses a multi-layer coating for drug release controllable stents comprising a base layer made of poly(ethylene-co-vinylacetate) or a styrenic rubber polymer, a second coating layer formed on the base layer using a biocompatible polymer and a drug ingredient, and a third coating layer formed on the second coating layer using a drug ingredient different from that of the second coating layer, and a method of manufacturing the same.

Examples of the biocompatible polymer include polyvinylalcohol, polyethyleneglycol, polylactide, polyglycolide, polylactide copolymers, polyethylene oxide, polydioxanone, polycaprolactone, polyphosphazene, polyanhydride, polyamino acid, cellulose acetate butyrate, cellulose triacetate, polyacrylate, polyacrylamide, polyurethane, polysiloxane, polyvinylpyrrolidone (PVP) and copolymers thereof, and examples of the drug ingredient of the second coating layer include cilostazol (6-[4-(1-cyclohexyl-1H-tetrazol-5-yl)butoxy]-3,4-dihydro-2(1H)-quinolinone, molecular formula: C₂₀H₂₇N₅O₂, molecular weight: 369.47) containing anti-platelet drugs, anti-thrombotic agents, antiproliferative agents, growth factors, antioxidants and radioactive compounds.

On the other hand, the present inventors filed and registered Korean Patent No. 0916750, titled ^{┌}Coating agent for drug releasing stent and method of manufacturing the same_{┘} .

Specifically the above patent is directed to a coating agent for a drug releasing stent containing 400 mg of polyurethane (PU), 21 ml of tetrahydrofuran (THF), 5%, 10%, 20% and 30% Pluronic F-127, and 5% bioactive taxol.

### SUMMARY OF THE INVENTION

Accordingly, the present invention has been made keeping in mind the above problems occurring in the related art, and an object of the present invention is to provide a method of manufacturing a coating agent for a drug releasing stent and a coating agent for a drug releasing stent manufactured thereby, in which a drug containing gemcitabine as an anti-cancer agent is prepared, and the drug thus prepared is applied on a stent so that the rate of release of the drug applied on the stent may be continuously maximized to improve anti-cancer effects.

In order to accomplish the above object, the present invention provides a method of manufacturing a coating agent for a drug releasing stent, including (1) dissolving polyurethane in tetrahydrofuran thus obtaining a first solution, (2) dissolving pluronic F-127 in tetrahydrofuran thus obtaining a second solution, (3) dissolving a gemcitabine compound in ethanol thus obtaining a third solution, (4) mixing the first solution, the second solution and the third solution obtained in (1) to (3) thus preparing a solution mixture, (5) applying the solution mixture obtained in (4) on a stent coated with a Teflon film, (6) drying the stent obtained in (5) for a predetermined period of time and then immersing the stent in a polyurethane solution in tetrahydrofuran, and (7) removing the stent immersed in (6) from the polyurethane solution and then drying the stent.

In (1), tetrahydrofuran may be used in an amount of 7 ~ 9 ml.

In (2), the amount of pluronic F-127 may be set in the range of 8 ~ 22 wt%.

In (3), the gemcitabine compound may be gemcitabine-hydrochloride (HCl), and the ethanol may be 1 ml of 75% ethanol (in H₂O, v/v).

In (4), mixing may be performed using ultrasonic waves. In (6) and (7), drying may be performed for 4 ~ 6 hr.

In (6), the stent may be immersed once in the polyurethane solution in tetrahydrofuran.

### BRIEF DESCRIPTION OF THE DRAWINGS

The above and other objects, features and advantages of the present invention will be more clearly understood from the following detailed description taken in conjunction with the accompanying drawings, in which:
FIG. 1 illustrates a flowchart of a process of manufacturing a coating agent for a drug releasing stent according to the present invention;
FIGS. 2 and 3 illustrate graphs of the results of a release test;
FIG. 4 illustrates the expression of the TNF-α gene depending on the time (week) of the coating agent for a drug releasing stent according to the present invention;
FIG. 5 illustrates the expression of the IL-1β gene depending on the time (week) of the coating agent for a drug releasing stent according to the present invention;
FIG. 6 illustrates the expression of the IL-12 gene depending on the time (week) of the coating agent for a drug releasing stent according to the present invention;
FIG. 7 illustrates the activation (phosphorylation) of the p38 MAPK depending on the time (week) of the coating agent for a drug releasing stent according to the present invention; and
FIG. 8 illustrates the effect of the drug released from the coating agent for a drug releasing stent according to the present invention on killing cancer cells depending on the time (week).

### DESCRIPTION OF SPECIFIC EMBODIMENTS

Hereinafter, embodiments of the present invention will be more clearly understood with reference to the appended drawings.

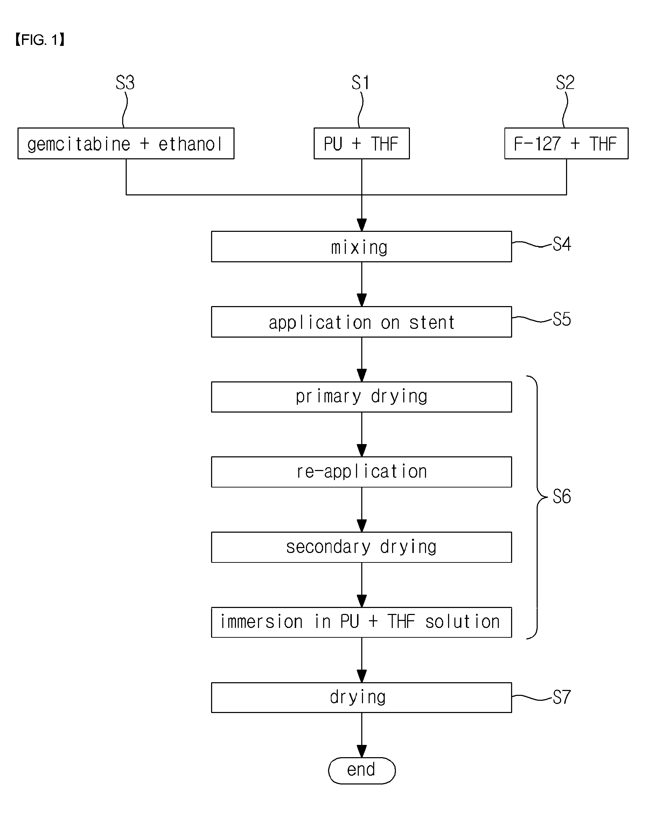

FIG. 1 illustrates a flowchart of a process of manufacturing a coating agent for a drug releasing stent according to the present invention.

As illustrated in FIG. 1, the method of manufacturing the coating agent for a drug releasing stent according to the present invention includes dissolving polyurethane in tetrahydrofuran thus obtaining a first solution (S1), dissolving pluronic F-127 in tetrahydrofuran thus obtaining a second solution (S2), dissolving a gemcitabine compound in ethanol thus obtaining a third solution (S3), mixing the first solution, the second solution and the third solution obtained in S1 to S3 thus preparing a solution mixture (S4), applying the solution mixture obtained in S4 on a stent coated with a Teflon film (S5), drying the stent of S5 for a predetermined period of time and then immersing it in a polyurethane solution in tetrahydrofuran (S6); and removing the stent immersed in S6 from the polyurethane solution and then drying it (S7).

In S1, polyurethane is dissolved in 7 ~ 9 ml of tetrahydrofuran (THF), exactly 8.5 ml of THF.

In S2, pluronic F-127 is dissolved in 1 ml of THF. The amount of pluronic F-127 is set in the range of 8 ~ 22 wt%.

More exactly, pluronic at 0%, 8%, 10%, 12%, 15%, and 22% may be used.

In S3, the gemcitabine compound is gemcitabine-HCl, and is dissolved in 1 ml of 75% ethanol (in H₂O, v/v).

In S4, these solutions are mixed using ultrasonic waves. In S6 and S7, drying is performed for 4 ~ 6 hr, exactly 5 hr.

In S6, the stent is immersed once in the polyurethane solution in THF.

The coating agent for a drug releasing stent manufactured via the above procedures is composed of polyurethane, THF, pluronic F-127, gemcitabine-HCl, and ethanol, which are mixed together, and is applied on the Teflon film and dried, thus completing a test sample, which is then subjected to the following tests to evaluate anti-cancer efficacy.

### [Test Example 1]

This test example pertains to release of the drug release coating agent according to the present invention.
(a) A Teflon film coated with the drug release coating agent is immersed in 2 ml of PBS.
(b) After initial 6 hr, the PBS is replaced.
(c) Subsequently, the PBS is replaced at intervals of 24 hr.
(d) Whenever PBS is replaced, measurement is performed at 266 nm using the obtained PBS using a spectrophotometer.

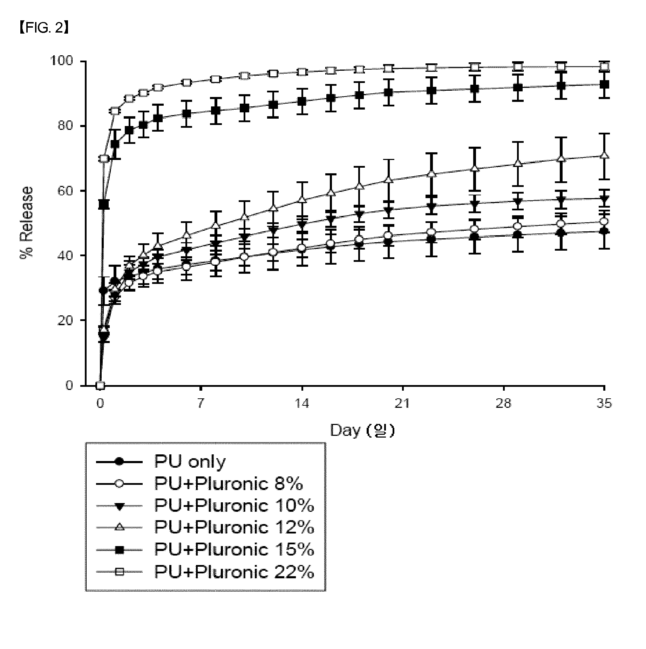

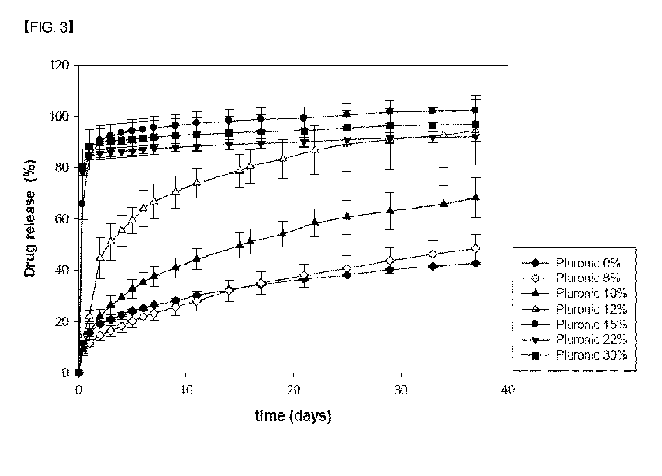

FIGS. 2 and 3 are graphs illustrating the results of the above release test.

Specifically, FIG. 2 illustrates the release of gemcitabine-HCl from the Teflon film, and FIG. 3 illustrates the release of gemcitabine-HCl from the bulk film.

The average amount of contained gemcitabine-HCl of the Teflon film was 7.78 **±** 0.05 mg/cm².

As is apparent from the release data depending on the concentration of pluronic in the mixture in which the final polymer concentration was fixed using polyurethane, in the case of the sample containing pluronic 12%, the amount of gemcitabine-HCl released per time was comparatively superior.

However, pluronic 0%, pluronic 8%, and pluronic 10% released a smaller amount per time, and the Teflon films containing pluronic 15% and pluronic 22% resulted in very large initial release, and thus their release curves were not regarded as ideal.

Compared to the release pattern (FIG. 3) of a film in which, as initial testing of this test, the same polymer mixing ratio was applied without using a Teflon film and the average amount of contained gemcitabine-HCl was 12.47 mg/cm², the above release trend was represented at pluronic 12%.

Thereby, pluronic 12% could be evaluated to more effectively release the drug.

### [Test Example 2]

By incubating human cholangiocarcinoma cells, namely, Sk-CHA-1, a molecular cytology test was conducted.

### (1) Method

### A. Cell Line

Sk-CHA-1 cells were incubated in Dulbecco's modified Eagle's medium (Sigma, St. Louis), 10% CO₂/90% O₂ containing 20% fetal bovine serum (GIBRO-BRL, Grand Island, NY, USA) and antibiotics (100 U/ml penicillin and 100 g/ml streptomycin).

Gemcitabine (GEM) released from the film over time (week) was added to cell lines and stimulated, and total RNA was extracted from respective cell lines and then c-DNA was synthesized.

Used as target cytokine genes expressed by gemcitabine were Tumor Necrosis Factor-α, Interleukin-1β, and Interleukin-12.

RT-PCR was performed using the primer and thus specific gene expression was measured.

The activation (phosphorylation) of p38-kinase known to be MAPK as a protein sample which lysed respective cell lines was evaluated via western blot.

### B. Gene Analysis

Gene sequence of the PCR primer used was as follows.

**[Table 1]**

| Gene Name | Primer | Sequence |
|---|---|---|
| Tumor Necrosis Factor-α | Sense | 5'-AGAAGGAAACAGACCACAGAC-3' |
| | Anti Sense | 5'-GGGAAAGAATCATTCACCCA-3' |
| Interleukin-1β | Sense | 5'-AGAAGCTTCCACCAATACTC-3' |
| | Anti Sense | 5'-AGCACCTAGTTGTAAGGAAG-3' |
| Interleukin-12 | Sense | 5'-AAGGAGGCGAGGTTCTAAGC-3' |
| | Anti Sense | 5'-GTACTCCCAGCTGACCTCCA-3' |

RT-PCR procedures including predenaturation at 94**°C** for 10 min, denaturation at 94**°C** for 60 sec, annealing at 62**°C** for 30 sec, and extension at 72**°C** for 45 sec were repeated 36 times, after which last extension at 72**°C** for 10 min was carried out, followed by performing electrophoresis (agarose 5% gel), and a band was measured under a UV lamp.

The internal standard material used was GAPDH (glyceraldehyde 3-phosphate dehydrogenase; 5'-ACCACAGTCCATGCCATCAC-3' (S primer)/5'-TCCACCACC CTGTTGCTGTA-3' (AS primer)).

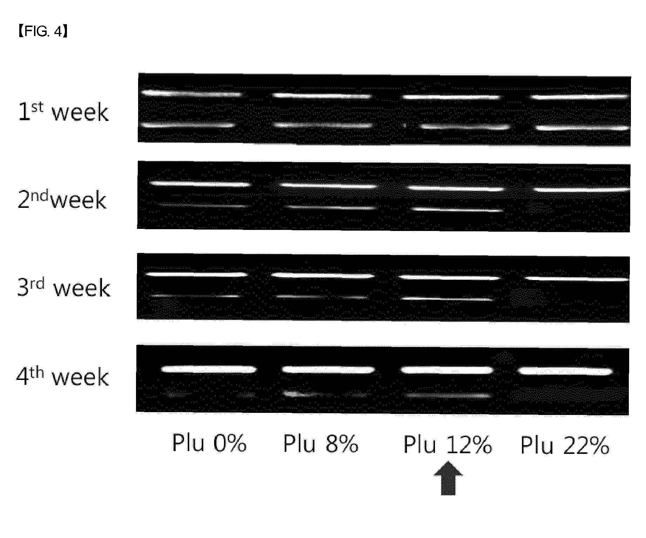

The results are shown in FIGS. 4 to 6.

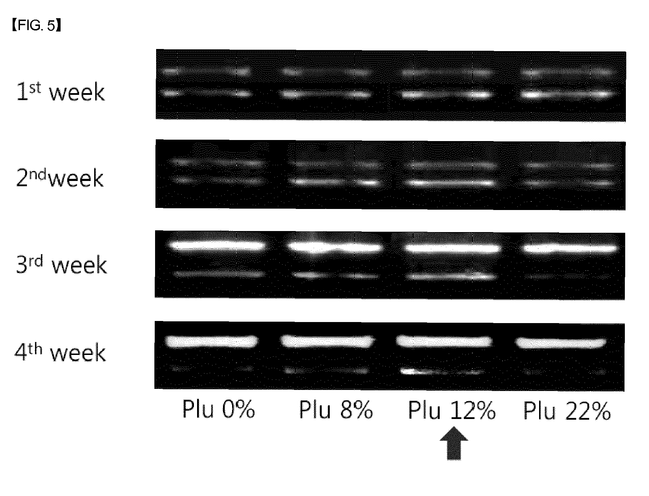

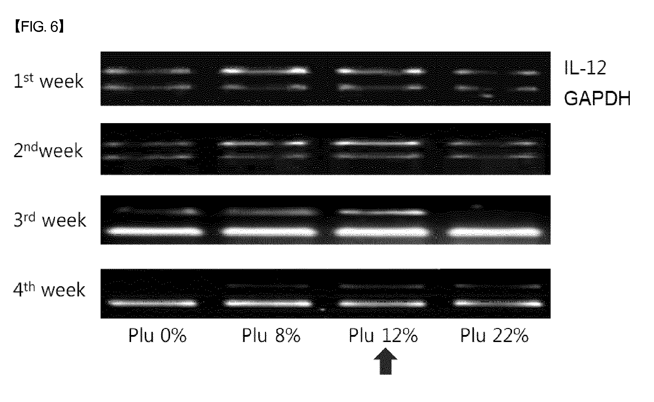

FIG. 4 illustrates the expression of the TNF-α, gene depending on the time (week) of the coating agent for a drug releasing stent according to the present invention, FIG. 5 illustrates the expression of the IL-1β gene depending on the time (week) of the coating agent for a drug releasing stent according to the present invention, and FIG. 6 illustrates the expression of the IL-12 gene depending on the time (week) of the coating agent for a drug releasing stent according to the present invention.

As illustrated in FIGS. 4 to 6, the drug was continuously released at pluronic 0%, pluronic 8%, and pluronic 12%. Particularly, in the case of pluronic 12% (Plu 12%), the expression of all the cytokine genes, namely, the TNF-α gene, the IL-1β gene, and the IL-12 gene, was detected even after the 4^{th} week, resulting in the longest expression.

### C. Protein Analysis (Activation of p38 MAPK)

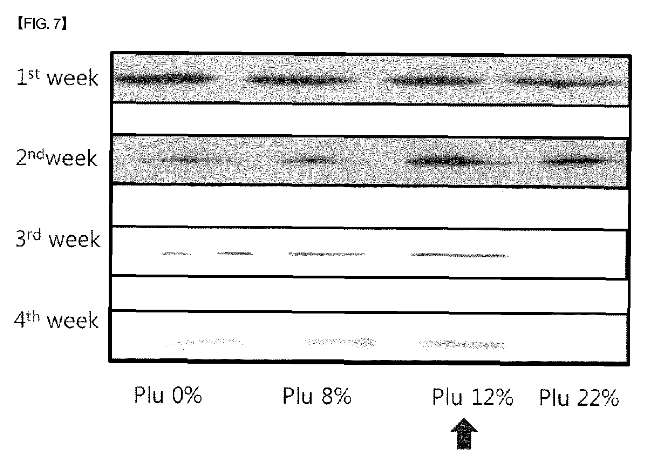

FIG. 7 illustrates the activation (phosphorylation) of p38 MAPK depending on the time (week) of the coating agent for a drug releasing stent according to the present invention.

As illustrated in FIG. 7, the drug was continuously released at pluronic 0%, pluronic 8%, and pluronic 12%. Particularly, in the case of pluronic 12% (Plu 120), phosphorylation of p38 was apparently observed after the 4^{th} week, resulting in the longest expression.

### [Test Example 3]

Cell death was tested by incubating human cholangiocarcinoma cells, namely Sk-CHA-1.

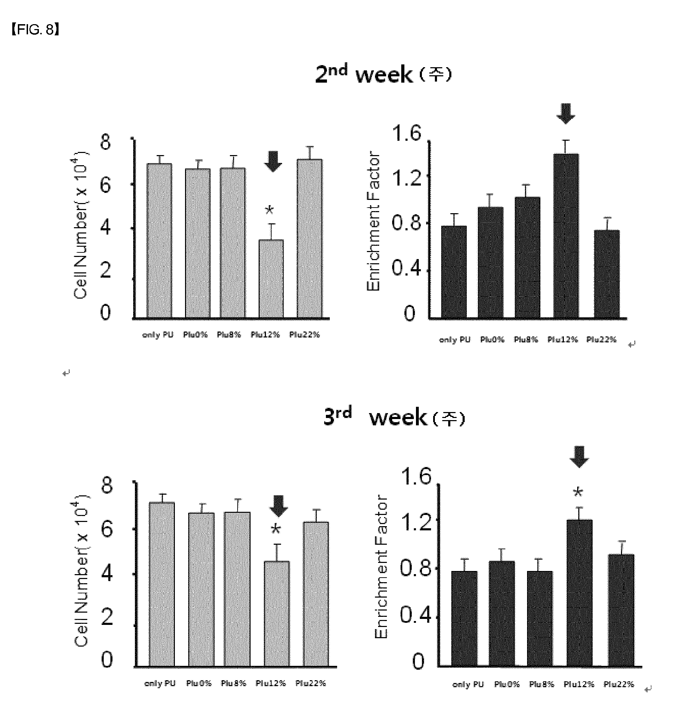

FIG. 8 illustrates the effect of the drug released from the coating agent for a drug releasing stent according to the present invention on killing cancer cells depending on the time (week).

The left graphs show the control treated with only PBS, and the right graphs show the test groups treated with pluronic 0%, pluronic 8%, pluronic 12%, and pluronic 22% according to the present invention.

Sk-CHA-1 cells were incubated in Dulbecco's modified Eagle's medium (Sigma, St. Louis), 10% CO₂/90% O₂ containing 20% fetal bovine serum (GIBRO-BRL, Grand Island, NY, USA) and antibiotics (100 U/ml penicillin and 100 g/ml streptomycin).

Gemcitabine released from the film over time (week) was added to cell lines. After 24 hr, the effects thereof on killing the cells were observed.

The cell death rate was determined by quantitatively measuring and comparing the cell numbers and the DNA fraction amounts in the medium after cell death.

As illustrated in FIG. 8, the drug was continuously released at pluronic 0%, pluronic 8%, and pluronic 12%. Particularly, pluronic 12% (Plu 12%) represented the highest death rate even after the 3^{rd} week, and the cell death was maximized because of continuous drug release.

In the release test of gemcitabine in the reaction system in which the total polymer amount was fixed, excellent release results were obtained at pluronic 12%, from which the present invention is regarded as effective in inhibiting and killing cancer cells.

As described hereinbefore, the present invention provides a method of manufacturing a coating agent for a drug releasing stent and a coating agent for a drug releasing stent manufactured thereby. According to the present invention, the rate of release of an anti-cancer drug applied on a stent can be continuously and maximally improved, thus maximizing anti-cancer efficacy.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method of manufacturing a coating agent for a drug releasing stent, comprising:
(1) dissolving polyurethane in tetrahydrofuran, thus obtaining a first solution;
(2) dissolving pluronic F-127 in tetrahydrofuran, thus obtaining a second solution;
(3) dissolving a gemcitabine compound in ethanol, thus obtaining a third solution;
(4) mixing the first solution, the second solution and the third solution obtained in (1) to (3), thus preparing a solution mixture;
(5) applying the solution mixture obtained in (4) on a stent coated with a Teflon film;
(6) drying the stent obtained in (5) for a predetermined period of time and then immersing the stent in a polyurethane solution in tetrahydrofuran; and
(7) removing the stent immersed in (6) from the polyurethane solution and then drying the stent.

2. The method of claim 1, wherein in (1), tetrahydrofuran is used in an amount of 7 ~ 9 ml.

3. The method of claim 1, wherein in (2), an amount of pluronic F-127 is set in a range of 8 ~ 22 wt%.

4. The method of claim 1, wherein in (3), the gemcitabine compound is gemcitabine-hydrochloride (HCl), and the ethanol is 1 ml of 75% ethanol (in H₂O, v/v).

5. The method of claim 1, wherein in (4), the mixing is performed using ultrasonic waves.

6. The method of claim 1, wherein in (6) and (7), the drying is performed for 4 ~ 6 hr.

7. The method of claim 1, wherein in (6), the stent is immersed once in the polyurethane solution in tetrahydrofuran.

8. A coating agent for a drug releasing stent, manufactured using the method of any one of claims 1 to 7.
